(19) Europäisches Patentamt
European Patent Office
Office européen des brevets

(11) Veröffentlichungsnummer: **0 003 750**
**B1**

(12) **EUROPÄISCHE PATENTSCHRIFT**

(45) Veröffentlichungstag der Patentschrift: **15.04.81**

(51) Int. Cl.³: **A 61 K 31/66** //A61K31/185

(21) Anmeldenummer: **79100177.9**

(22) Anmeldetag: **22.01.79**

(54) **Salze von Dithiodialkansulfonsäuren zur Verwendung bei der cytostatischen Therapie mit Alkylantien.**

(30) Priorität: **17.02.78 DE 2806866**

(43) Veröffentlichungstag der Anmeldung:
**05.09.79 Patentblatt 79/18**

(45) Bekanntmachung des Hinweises auf die Patenterteilung:
**15.04.81 Patentblatt 81/15**

(84) Benannte Vertragsstaaten:
**BE CH FR GB IT LU NL SE**

(56) Entgegenhaltungen:
**Keine**

(73) Patentinhaber: **Asta-Werke Aktiengesellschaft Chemische Fabrik**
**Artur-Ladebeck-Strasse 128 - 152**
**D-4800 Bielefeld 14 (DE)**

(72) Erfinder: **Brock, Norbert, Prof. Dr.**
**Am Rehhagen 10**
**D-4800 Bielefeld 1 (DE)**
Erfinder: **Stekar, Jurij, Dr.**
**Asta-Strasse 29**
**D-4800 Bielefeld 14 (DE)**

(74) Vertreter: **Redies, Bernd, Dr. rer. nat. et al,**
**Redies, Redies, Türk & Gille, Patentanwälte**
**Brucknerstrasse 20**
**D-4000 Düsseldorf 13 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 003 750 B1

# Salze von Dithiodialkansulfonsäuren zur Verwendung bei der cytostatischen Therapie mit Alkylantien

Bei alkylierenden Cytostatika (nachfolgend Alkylantien-Cytostatika bezeichnet) wie Melphalan, Cyclophosphamid, Trofosfamid, Ifosfamid, Sufosfamid, Chlorambucil, Busulfan, Triäthylenthiophosphamid oder Triaziquon und insbesondere den 2-Oxo-1,3,2-oxazaphosphorinanen Cyclophosphamid, Trofosfamid, Ifosfamid und Sufosfamid treten als unerwünschte Nebenwirkung schwere Reizungen der Niere, Harnwege und/oder der Harnblase des behandelten Patienten auf. Bekannterweise treten nach erstmaliger Schädigung diese Nebenwirkungen besonders leicht auf. Diese unerwünschte Nebenwirkung kann dabei so stark sein, daß die Therapie des an Krebs erkrankten Patienten zeitweise unterbrochen oder sogar ganz unmöglich wird. Ein wesentlicher Erfolg solcher Cytostatika liegt wegen der raschen Resistenzentwicklung des Krebstumors auch in der sogenannten Stoßtherapie, bei der die Cytostatika bei ihrer ersten Applikation in im Verhältnis zu ihrer Toxizität hoher Einzeldosis verabreicht werden, um eine möglichst hohe Initialkonzentration des Cytostatikums am Tumorgewebe zu erreichen. Danach werden die Cytostatika in geringeren Dosen häufig über lange Zeiträume verabreicht. Es ist bekannt, daß die geschilderte unerwünschte Nebenwirkung durch im Körper des Patienten gebildete Metaboliten der Cytostatika verursacht werden. Die Nebenwirkung tritt schon bei länger bekannten Alkylantien wie den unter der bekannten Bezeichnung Endoxan® oder Cyclophosphamid bekanntgewordenen 2 - (N,N - Bis - (2 - chloräthyl) - amino) - 2 - oxo-1,3,2 - oxazaphosphorinan und unter dem unter der Bezeichnung Ixoten® oder Trofosfamid bekanntgewordenen 2 - (N,N - Bis - (2 - chloräthyl) - amino) - 3 - (2 - chloräthyl) - 2 - oxo -1,3,2 - oxazaphosphorinan häufig auf. Noch höhere Bedeutung hat diese Nebenwirkung, wenn man hoch cytostatisch wirksame und gleichzeitig weniger toxische Alkylantien-Cytostatika wie das Ifosfamid verabreicht. Die therapeutischen Einsatzmöglichkeiten, die aufgrund der geringen Toxizität gegeben sind, werden durch diese Nebenwirkung entscheidend eingeschränkt. Es wurde sogar beobachtet, daß aufgrund solcher Reizungen der Blase sich Blasenkrebs bildete.

Man hat viele Versuche unternommen, diese schädliche und unerwünschte Nebenwirkung der Alkylantien-Cytostatika zu beseitigen oder zumindest zu lindern, da auf den Einsatz der Alkylantien in der Behandlung von Krebskrankheiten nicht mehr verzichtet werden kann und ein vorzeitiger Abbruch der Therapie zu einer schweren Schädigung oder zu einem frühen Tod des behandelten Patienten führt. So soll durch erhöhte Flüssigkeitsaufnahme gegebenenfalls in Verbindung mit die Urinbildung fördernden Mitteln ein möglichst rascher Durchfluss von Metaboliten der Cytostatika enthaltendem Urin durch Niere, Harnwege und Blase erreicht und die Bildung zu hoher Konzentrationen der Metaboliten insbesondere in der Blase vermieden werden. Diese sogenannte Hydratation wird im allgemeinen mit einer Alkalisierung des Urins z.B. mit dem unter der Bezeichnung Uralyt®—U im Handel befindlichen Hexakaliumhexanatriumpentacitrat-Hydrat-Komplex und insbesondere mit der Einführung von Lösungen von Mercaptogruppen enthaltenden Verbindungen in die Blase durch Katheter verbunden. Von solchen Mercaptogruppen enthaltenden Verbindungen nahm man an, daß die Mercaptogruppe mit den alkylierenden Gruppen reagiert und so inaktiviert. Als solche Verbindung wurde insbesondere N-Acetylcystein und Cystein verwendet. Die Erfolge sind jedoch nur begrenzt, insbesondere bei Einsatz der Cytostatika in sehr hohen Dosen. Außerdem sind Blasenspülungen für den Patienten beschwerlich und bei Verabreichung der Cytostatika über längere Zeiträume praktisch kaum durchführbar. Schließlich sind hierdurch höher gelegene Bereiche der Harnwege überhaupt nicht erreichbar.

Eine der ersten Arbeiten, die sich mit dem Einsatz von SH-Verbindungen zur generellen Detoxifizierung bei der Alkylantien-Therapie befaßt, ist T. A. Connors, Europ. J. Cancer *2*, 293 bis 395 (1966). Ein Erfolg dieser Versuche blieb aus, da mit den eingesetzten SH-Verbindungen gleichzeitig der Antitumoreffekt der Alkylantien vermindert wurde (vgl. loc. cit. S. 300 und 303, vorletzter Satz).

Als mit der Einführung der Oxazaphosporine über das Auftreten von urotoxischen Nebenwirkungen (hämorrhagische Zysto-pyelonephritis) geklagt wurde, hat man versucht, SH-Verbindungen topisch, d.h. an den Ort der Wirkung in die Harnblase zu bringen. Diese Instillation von Acetylcystein gehört zur Standardprophylaxe gegenüber urotoxischen Nebenwirkungen bei der hochdosierten Applikation von Cyclophosphamid und Ifosfamid (vgl. z.B. HOEFER-JANKER, SCHEEF, GÜNTHER, HÜLS, Med. Welt *26* 972, 1975; DRINGS, FRITSCH, Verh. Dtsch. Ges. inn. Med. *78*, 166, 1972; COHEN, CREAVEN, TEJADA, HANSEN, MUGGIA, MITTELMANN, SELAWRY, Cancer Chemother. Rep. Part 1, 59, 751, 1975; CREAVEN, ALLEN, COHEN, NELSON, Cancer Treatm. Rep. *60*, 445, 1976; und PRIMACK, J. Nat. Cancer Inst. 47, 223 (1971).

Die Instillation von SH-Gruppen in die Harnblase konnte das Problem der generellen Detoxifizierung jedoch nicht lösen. Die Wirksamkeit der verwendeten SH-Verbindungen ist auf die Harnblase beschränkt. Die Praktikabilität dieser Methode (Applikation mittels Katheter) ist wenig positiv zu beurteilen. Die klinische Wirksamkeit dieser umständlichen Prophylaxe ist keineswegs befriedigend (vgl. FALKSON, Suid-Afrikaanse Kankerbulletin *15*, 97, 1971).

In der deutschen Patentanmeldung P 27 56 018.5 vom 14.12. 1977 wird nun beschrieben, daß die geschilderte, seit langem bekämpfte schädliche Nebenwirkung von alkylierend wirkenden Cytostatika auf die Harnwege und Blase der hiermit behandelten Menschen durch Anwendung der lange bekannten pharmakologisch annehmbaren Salze von Mercaptoalkansulfonsäuren der allgemeinen Formel

$$HS—alk—SO_3H,$$

worin alk ein geradkettiger oder verzweigter Alkylenrest mit 2 bis 6, insbesondere 2 bis 4 Kohlenstoffatomen ist, in bestimmter Weise im Verhältnis zur Verabreichung des Cytostatikums erreicht wird, und zwar innerhalb eines Zeitraumes von etwa 30 Minuten vor Verabreichung des Cytostatikums bis etwa 30 Minuten nach dessen Verabreichung und in einer Menge von mindestens 20% der zu verabreichenden bzw. verabreichten Menge des Cytostatikums bis höchstens der höchstverträglichen Dosis des Salzes der Mercaptoalkansulfonsäure.

Die Salze der Mercaptoalkansulfonsäuren zeigen jedoch vielfach nicht die gewünschte ideale Schutzwirkung, da sie einen vom Ausscheidungsverlauf der Alkylantien-Cytostatika und ihrer alkylierenden Metaboliten zu stark unterschiedlichen Ausscheidungsverlauf zeigen. Auch sind die Salze der Mercaptoalkansulfonsäuren nicht besonders stabil Sauerstoff gegenüber, so daß ihre Verarbeitung insbesondere zu peroral verabreichbaren Zubereitungsformen beträchtlich erschwert und deren Lagerungsstabilität stark vermindert ist. Gerade diese Applikationsform erwies sich jedoch als besonders notwendig, da eine aufgrund des unterschiedlichen Abscheidungsverlaufs der zusammenwirkenden Produkte wiederholte Applikation des Salzes der Mercaptoalkansulfonsäure mit den bekannten, zumeist sehr schlechten Venenverhältnissen nicht auf intravenösem Weg erfolgen kann.

Überraschenderweise wurde nun gefunden, daß die geschilderte schädliche Nebenwirkung von alkylierend wirkenden Cytostatika auf die Harnwege und die Blase der hiermit behandelten Menschen durch Anwendung von pharmakologisch annehmbaren Salzen der bekannten Dithiodialkansulfonsäuren der allgemeinen Formel

$$HO_3S—alk—SS—alk—SO_3H,$$

worin alk ein geradkettiger oder verzweigter Alkylenrest mit 2 bis 6, vorzugsweise 2 bis 4 Kohlenstoffatomen ist, verhindert wird. Vorzugsweise wird das Salz der Dithiodialkansulfonsäure in bestimmter Weise im Verhältnis zur Verabreichung des Cytostatikums eingesetzt, und zwar innerhalb eines Zeitraumes von etwa 120 Minuten vor Verabreichung bis etwa 120 Minuten nach Verabreichung des Cytostatikums und in einer Menge von mindestens 20% der zu verabreichenden bzw. verabreichten Menge des Cytostatikums bis höchstens der höchstverträglichen Dosis des Salzes der Dithiodialkansulfonsäure. Dieses Ergebnis ist darum überraschend, weil zur Erzielung der Schutzwirkung gegen die unerwünschte Nebenwirkung stets von Verbindungen mit freien Mercaptogruppen als Wirkgruppe ausgegangen wurde und bei ähnlich gelagerten Fällen bekannt ist, daß die Wirksamkeit von Mercaptoverbindungen bei Umwandlung in das entsprechende Disulfid praktisch verschwindet. Dementsprechend zeigt zwar das Cystein gegenüber den Salzen der Mercaptoalkansulfonsäuren noch eine deutliche, wenn auch geringere Schutzwirkung gegen Alkylantien-Cytostatika, das entsprechende Disulfid, nämlich das Cystin überhaupt keine solche Schutzwirkung mehr. Dies entspricht dem bekannten Umstand, daß organische Disulfide unter in vivo-Bedingungen nicht leicht reduzierbar sind (vgl. für das Disulfid System z.B. Bertho und Grassmann, Biochemisches Praktikum, 1936, S. 143). Auch bei anderen reduzierbaren Verbindungen mit physiologischer Wirksamkeit ist bekannt, daß die reduzierte Form unwirksam ist, wie z.B. bei der Ascorbinsäure/Desoxyascorbinsäure.

Das Salz der Dithiodialkansulfonsäure kann dabei in der gleichen oder in einer anderen üblichen Applikationsform als das Cytostatikum verabreicht werden. Z.B. kann das Cytostatikum intravenös, das Salz der Dithiodialkansulfonsäure dagegen peroral oder intraperitoneal appliziert werden. Die vorliegende Erfindung betrifft somit auch diese Applikationsformen, d.h. pharmazeutische Produkte, enthaltend ein pharmakologisch annehmbares Salz der Dithiodialkansulfonsäure als einzigen Wirkstoff neben üblichen Träger- und Zusatzstoffen oder zusammen mit einen Alkylans-Cytostatikum. Sowohl allein als auch zusammen mit dem Cytostatikum lassen sie sich leicht zu allen Applikationsformen, insbesondere auch peroral verabreichbaren Produkten verarbeiten.

Bevorzugt wird das Salz der Dithiodialkansulfonsäure mit dem Cytostatikum gleichzeitig verabreicht. Dies gilt insbesondere für die in der Stoßtherapie übliche hohe Initialdosis. Bevorzugt werden dann beide Verbindungen in einer einzigen Applikationseinheit verabreicht.

Als salzbildende Basen kommen zahlreiche Verbindungen in Betracht, wie sie z.B. auch für die Mercaptoalkansulfonsäuren bekannt sind [US—PS 2 694 732; DE—OS 1 629 629]. Besonders geeignet sind die Alkalisalze, insbesondere die Natriumsalze.

Unter den Salzen der Dithiodialkansulfonsäuren haben besonders gute Ergebnisse diejenigen der Dithiodiäthan-ß-sulfonsäure bzw. der 2,2'-Dithiodi-(äthansulfonsäure) der Formel

$$HO_3S—CH_2CH_2—SS—CH_2CH_2—SO_3H,$$

gegeben, die daher besonders bevorzugt sind. Ganz besonders geeignet ist hierbei das Dinatriumsalz der Dithiodiäthan-ß-sulfonsäure.

Um einen wirkungsvollen Schutz der mit den Cytostatika behandelten Patienten gegen die Nebenwirkung auf die Niere, Harnwege und Harnblase zu erreichen, genügt die Verabreichung schon so geringer Mengen wie 20% der Dosis des Cytostatikums. Dies gilt insbesondere bei niedrigeren Dosen des Cytostatikums. Bei höheren Dosen des Cytostatikums kann die schädliche Nebenwirkung mit 30% der Menge des Cytostatikums verhindert werden. Da die Nebenwirkung insbesondere bei der Verabreichung der Cytostatika in hohen Dosen von Bedeutung ist, wird die Untergrenze von 30% der Menge des Cytostatikums bevorzugt angewendet. Im Hinblick auf die sehr geringe Toxizität der pharmakologisch annehmbaren Salze der Dithiodialkansulfonsäuren ist die obere Grenze des Mengenbereichs für das Salz der Dithiodialkansulfonsäure von untergeordneter Bedeutung. Überraschend und wichtig ist, daß die cytostatische Wirksamkeit der Alkylantien nicht beeinträchtigt wird. Da im allgemeinen eine praktische Beseitigung der Nebenwirkung auch bei hohen Cytostatika-Dosen mit etwa gleichen Mengen des Salzes der Dithiodialkansulfonsäure erreicht werden, ist es bevorzugt, das Salz der Dithiodialkansulfonsäure in einer Menge entsprechend 30 bis 150% der Menge des bei Verabreichung der Dithiodialkansulfonsäure vor Verabreichung des Cytostatikums zu verabreichenden bzw. des verabreichten Cytostatikums anzuwenden.

Während die Salze der Dithiodialkansulfonsäuren in Verbindung mit allen Alkylantien eingesetzt werden können und hierbei die geschilderte schädliche und besonders unerwünschte Nebenwirkung verhindern, haben sie besondere Bedeutung in Verbindung mit den in großem Umfang zur Bekämpfung verschiedenster Krebskrankheiten eingesetzten 2-Oxo-1,3,2-oxazaphosphorinane Cyclophosphamid, Ifosfamid, Trofosfamid und Sufosfamid.

Die erfindungsgemäß angewendeten pharmakologisch annehmbaren Salze der Dithiodialkansulfonsäuren sind zum Teil bekannte Verbindungen [vgl. J.Org.Chem. *26* (1961) 1330]. Für therapeutische Zwecke sind solche Salze bislang nicht verwendet worden. Auch für die Verhinderung der geschilderten Nebenwirkung der Alkylantien-Cytostatika sind diese oder ähnliche Disulfid-Verbindungen bisher nicht eingesetzt worden. Bisher war man der Meinung, daß die die schädliche Nebenwirkung verursachenden Alkylantien bzw. Metaboliten der Alkylantien regional am Ort der Schädigung abgefangen werden und dabei Mercaptogruppen enthaltende Verbindungen dort (z.B. durch Instillation in die Blase der behandelten Patienten) eingesetzt werden müssen, damit diese ihre Wirkung entfalten können, und daß es auf alle Fälle Mercaptogruppen enthaltende Verbindunen sein müssen. Schließlich war man der Meinung, daß gerade die in Betracht gezogenen Metaboliten für die cytostatische Wirksamkeit der Alkylantien verantwortlich sind und die Mercaptogruppen der eingesetzten Verbindungen für die Schutzwirkung maßgeblich sind und daß diese Verbindungen dementsprechend nur so spät zur Anwendung gebracht werden dürfen, daß die cytostatische Wirksamkeit nicht negativ beeinflußt wird. Selbst bei Anwendung der bisher verwendeten Mercaptogruppen enthaltenden Verbindungen ist die hierbei erreichte Wirkung im allgemeinen nur beschränkt. Die geschilderten Nebenwirkungen können auch nicht angenähert verhindert werden. Bei entsprechenden Disulfiden war die Schutzwirkung überhaupt nicht gegeben. Es ist somit nicht nur überraschend, daß die erfindungsgemäß eingesetzten Salze der Dithiodialkansulfonsäuren überhaupt die Schutzwirkung ausüben und dazu in einem solchen Ausmaß, daß die geschilderte schädliche Nebenwirkung praktisch vollständig vermieden wird, sondern daß diese Verbindungen diese Wirkung auch bei oraler Verabreichung in vollem Umfang entfalten.

Die folgenden Beispiele dienen der weiteren Erläuterung der Erfindung, ohne sie allerdings hierauf zu beschränken.

*Anwendungsbeispiele*

1) Ein 27 Jahre alter Patient, der an einem Teratokarzinom des Hodens leidet, wird nach den Regeln der Therapie mit Bestrahlung und Ifosfamid (2 - [N - (2 - Chloräthyl) - amino] - 3-(2 - chloroäthyl) - 2 - oxo - 1,3,2 - oxazaphosphorinan) (5 × 60 mg/kg intravenös an 5 aufeinander folgenden Tagen) behandelt. Gleichzeitig wird der Patient sowohl oral als auch intravenös durch Flüssigkeitszufuhr, über den Tag verteilt, hydratisiert und durch Verabreichung von Hexakaliumhexanatriumpentacitrathydrat-Komplex alkalisiert. Trotz dieser Prophylaxe reagiert der Patient am 4. Tag mit einer makroskopisch erkennbaren Hämaturie, die zum sofortigen Absetzen der Therapie zwingt. Nach einer Erholungsphase von 14 Tagen wird die Therapie wiederholt. Diesmal wird gleichzeitig mit jeder Dosis von 60 mg/kg Ifosfamid 60 mg/kg 2,2′ - Dithiodi - (äthan - natriumsulfonat) intravenös appliziert. Der Patient wurde täglich auf Mikro- und Makrohämaturien sowie auf Ausscheidung von granulierten Zylindern und Eiweiß (im Urin) kontrolliert. Erythrozyten, Eiweiß und Zylinder waren zu keiner Zeit während der Behandlung im Urin nachweisbar.

2) An einem weiteren Patienten in analoger Situation wurde die Therapie zunächst mit der oralen Gabe von 2,2′-Dithiodi-(äthan-natriumsulfonat) (60 mg/kg) eingeleitet. Zwei Stunden später wurde Ifosfamid in einer Dosierung von 60 mg/kg appliziert. Das gleiche Therapieverfahren, also die Kombination einer oralen Vorgabe von 2,2′-Dithiodi-(äthan-natriumsulfonat) und nachfolgender intravenöser Gabe von Ifosfamid (60 mg/kg) wurde an 5 aufeinander folgenden Tagen durchgeführt. Auch hier wurden keinerlei Zeichen einer Nieren- und Blasenschädigung festgestellt. Hämaturie, Eiweiß und granulierte Zylinder waren während der Behandlung nicht nachweisbar.

Bei beiden vorstehend beschriebenen Behandlungen wurde eine mäßige Hydration (2 l pro Tag) durchgeführt. Die Alkalisierung sowie die lästige und beschwerliche Instillation von SH-Gruppen enthaltenden Verbindungen in die Blase mit Katheterismus entfielen bei diesen Patienten.

3) Ein weiterer Patient in analoger Situation war durch vorangegangene regionale Radiotherapie für Blasenschädigungen sensibilisiert. Erfahrungsgemäß kommt es bei solchen Patienten trotz aller Prophylaxemaßnahmen (Hydration, Alkalisierung, Blaseninstillation von SH-Gruppen enthaltenden Verbindungen) sehr leicht zu hämorrhagischer Zystitis. Auch bei diesem Patienten konnten Nieren- und Blasenschädigung durch Ifosfamid-Therapie (60 mg/kg Einzeldosis i.v. über 5 Tage) durch gleichzeitige Gabe von 60 mg/kg i.v. 2,2'-Dithiodi-(äthan-natriumsulfonat) vollständig verhütet werden.

4) Eine 48 Jahre alte Patientin, die an einem Ovarialkarzinom leidet, wird nach den Regeln der Therapie mit Bestrahlung und Ifosfamid [2 - (N - 2 - Chloräthylamino) - 3 - (2 - chloräthyl) - 2 - oxo - 1,3,2 - oxazaphosphorinan] (5 × 60 mg/kg intravenös an 5 aufeinander folgenden Tagen) behandelt. Trotz Hydratation und Alkalisierung des Urins kommt es zu einer Mikrohämaturie, die zum Absetzen der Therapie führt. Nach einer Erholungspause von 14 Tagen wird die Therapie wiederholt. Gleichzeitig mit jeder Dosis von 60 mg/kg Ifosfamid werden 35 mg/kg des Natriumsalzes der 2-Mercaptoäthansulfonsäure intravenös appliziert. 60, 120 und 180 Minuten nach der intravenösen Injektion erhält die Patientin je 45 mg 2,2'-Dithiodi-(äthan-natriumsulfonat). Die Patientin wird täglich auf Mikro- und Makrohämaturien und Eiweißausscheidung im Urin kontrolliert. Es waren keinerlei Anzeichen einer Hämaturie oder Eiweißausscheidung feststellbar. Auch eine Prüfung des Urins auf zytotoxische Wirksamkeit zu verschiedenen Zeiten nach Ifosfamid-Gabe verlief negativ. Zytotoxisch aktive Metaboliten waren in dem Urin nicht mehr nachweisbar.

Eine gleiche Schutzbehandlung wurde auch bei Patienten mit anderen Tumorleiden (kleinzelliges Bronchialkarzinom, Pankreaskarzinom) mit gleichem gutem Erfolg wiederholt.

*Arzneimittelbeispiele*

5) Ifosfamid (2 - (N - (2 - Chloräthyl) - amino)-3 - (2 - chloroäthyl) - 2 - oxo - 1,3,2 - oxazaphosphorinan und 2,2' - Dithiodi - (äthan-natriumsulfonat) werden im Gewichtsverhältnis 1:1 in einem sterilen Mischer unter aseptischen Bedingungen homogen gemischt und so in Injektionsflaschen gefüllt, daß diese auf 10 ml Injektionsflüssigkeit 500 mg von jedem der beiden Wirkstoffe enthalten.

6) Es werden Manteltabletten von 0,600 mg Gewicht hergestellt, wobei der Kern mit 200 mg Gesamtgewicht 50 mg Trofosfamid (2 (N,N-Di - (2 - chloräthyl) - amino) - 3 - (2 - chloräthyl) - 2 - oxo - 1,3,2 - oxazaphosphorinan und 50 mg 2,2' - Dithiodi - (äthan - natriumsulfonat) als Wirkstoffe sowie 62,3 mg Maisstärke, 17,8 mg Milchzucker, 0,9 mg Gelatine, 12,0 mg Talkum, 4,0 mg Magnesiumstearat und 3,0 mg Aerosil als Hilfsstoffe enthält und der Mantel mit 400 mg Gesamtgewicht 154,0 mg Maisstärke, 115,0 mg Milchzucker, 106,5 mg Calciumphosphat, 5,7 mg Gelatine, 2,8 mg Glycerin, 12,0 mg Talkum und 4,0 mg Magnesiumstearat enthält.

0,4 kg Maisstärke und 0,178 kg Milchzucker werden mit 0,18 kg einer 5%igen wässrigen Gelatinelösung angefeuchtet, durch ein 2-mm-Sieb granuliert und getrocknet. Anschließend gibt man dieses Granulat, 0,5 kg Trofosfamid, 0,5 mg 2,2'-Dithiodi-(äthan-natriumsulfonat), 0,223 kg Maisstärke, 0,120 kg Talkum, 0,030 kg Aerosil und 0,040 kg Magnesiumstearat durch ein 1-mm-Sieb. Diese Masse wird homogen gemischt und auf einer Rundläuferpresse zu Kernen von 9 mm Durchmesser, einem Wölbungsradius von 7,5 mm und einem Gewicht von 200 mg verpreßt.

Aus 0,057 kg Gelatine und 0,028 kg Glycerin wird eine 5%ige wässrige Gelatinelösung hergestellt und die homogene Mischung aus 1,540 kg Maisstärke, 1,150 kg Milchzucker und 1,065 kg Calciumphosphat mit dieser Lösung angefeuchtet. Anschließend wird durch ein 2-mm-Sieb granuliert und getrocknet. Diese Masse wird schließlich mit 0,120 kg Talkum und 0,040 kg Magnesiumstearat durch ein 1-mm-Sieb gegeben und homogen gemischt, um das preßfertige Mantelgranulat zu ergeben.

Auf einem Kilian-Prescoter werden die Kerne mit dem Mantelgranulat zu Mantelbletten von 600 mg Gewicht, 12 mm Durchmesser und einem Wölbungsradius von 12,5 mm verarbeitet.

7) 20,0 kg 2,2'-Dithiodi-(äthan-natriumsulfonat) werden in Wasser für Injektionszwecke auf eine Lösung von 100,0 l aufgefüllt. Die 20%ige wässrige Lösung ist klar und farblos und wird in 5 ml Ampullen gefüllt, so daß jede Ampulle 1 g Wirkstoff enthält. Nach Sterilisierung in üblicher Weise sind die Ampullen gebrauchsfertig.

8) 7,500 kg 2,2'-Dithiodi-(äthan-natriumsulfonat), 0,900 kg Milchzucker, 0,900 kg Calciumphosphat und 1,200 kg Maisstärke werden gemischt, mit einer 5%igen Gelatinelösung (1,5 l enthaltend 0,075 kg Gelatine) angeteigt und durch ein 2-mm-Sieb gegeben. Nach dem Trocknen werden dieses Granulat und 0,375 kg Plasdone XL, 0,225 kg Talkum und 0,075 kg Magnesiumstearat durch ein 1-mm-Sieb gegeben und homogen vermischt. Die Masse wird zu Kernen von 750 mg Gewicht, 13 mm Durchmesser und einem Wölbungsradius von 18 mm verpreßt.

170,0 g Milchzucker, 17,0 g Polyglycol 6000 und 17,0 g Tween 20 werden in 1021,7 g gereinigtes Wasser gelöst, anschließend 176,8 g Titandioxid und 170,0 g Talkum suspendiert. Nach Einrühren von 127,5 g Eudragit E 30 d wird die Suspension auf die Kerne aufgetragen, so daß jeder Kern 24,0 mg Filmhülle trägt.

**Patentansprüche**

1. Pharmakologisch annehmbare Salze einer Dithiodialkansulfonsäure der allgemeinen Formel

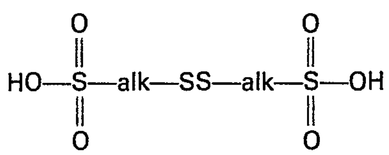

worin alk ein geradkettiger oder verzweigter Alkylenrest mit 2 bis 6 Kohlenstoffatomen ist, zur Verwendung bei der zytostatischen Therapie mit Alkylantien.

2. Salze gemäß Anspruch 1, dadurch gekennzeichnet, daß als alk der 1,2-Äthylen-Rest ist.

3. Als Verbindung gemäß Anspruch 1 und 2 das Natriumsalz der 2,2'-Dithiodi-(äthansulfonsäure).

4. Pharmazeutische Produkte, enthaltend ein pharmakologisch annehmbares Salz einer Dithiodialkansulfonsäure der allgemeinen Formel

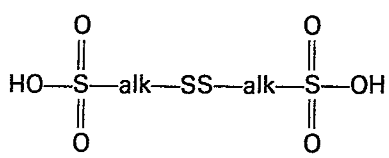

worin alk ein geradkettiger oder verzweigter Alkylenrest mit 2 bis 6 Kohlenstoffatomen ist, und ein Alkylans-Cytostatikum.

5. Pharmazeutische Produkte gemäß Anspruch 4, enthaltend ein Salz der 2,2'-Dithiodi-(äthansulfonsäure).

6. Pharmazeutische Produkte gemäß Anspruch 5, enthaltend das Dinatriumsalz der 2,2'-Dithiodi-(äthansulfonsäure).

7. Pharmazeutische Produkte gemäß Ansprüchen 4 bis 6, enthaltend als Alkylans-Cytostatikum 2 - [N,N - Bis - (2 - chloräthyl) - amino]-3 - (2 - chloräthyl) - 2 - oxo - 1,3,2 - oxazaphosphorinan (Trofosfamid), 2 - [N - (2 - chloräthyl)-amino] - 3 - (2 - chloräthyl) - 2 - oxo - 1,3,2-oxazaphosphorinan (Ifosfamid), 2 - [N,N - Bis-(2 - chloräthyl) - amino] - 2 - oxo - 1,3,2-oxazaphosphorinan (Cyclophosphamid) oder 2-(2 - Mesyloxyäthylamino) - 3 - (2 - chloräthyl)-2 - oxo - 1,3,2 - oxazaphosphorinan (Sufosfamid).

8. Pharmazeutische Produkte gemäß Ansprüchen 4 bis 7, enthaltend das Salz der Dithiodialkansulfonsäure und das Alkylans-Cytostatikum in einem Gewichtsverhältnis von mindestens 0,2:1.

**Claims**

1. Pharmacologically acceptable salts of a dithiodi-(alkane sulfonic acid) of the general formula

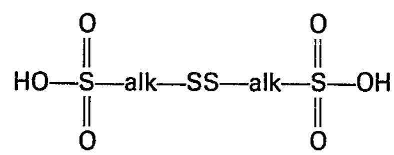

wherein alk is a straight or branched alkylene group having from 2 to 6 carbon atoms, for the use in the cytostatic therapy with alkylating agents.

2. Salts according to claim 1 characterized in that alk is the 1,2-ethylene group.

3. A compound according to claims 1 and 2 the sodium salt of 2,2'-dithiodi-(ethane sulfonic acid).

4. Pharmaceutical products containing a pharmaceutically acceptable salt of a dithiodi-(alkane sulfonic acid) of the general formula

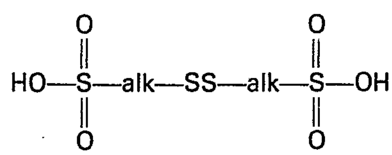

wherein alk is a straight or branched alkylene group having from 2 to 6 carbon atoms, and a cytostatically active alkylating agent.

5. Pharmaceutical products according to claim 4 containing a salt of the 2,2'-dithiodi-(ethane sulfonic acid).

6. Pharmaceutical products according to claim 5 containing the disodium salt of 2,2'-dithiodi-(ethane sulfonic acid).

7. Pharmaceutical products according to claims 4 to 6 containing as cytostatically active alkylating agent 2 - (N,N - bis - (2 - chloroethyl)-amino) - 3 - (2 - chloroethyl) - 2 - oxo - 1,3,2-oxazaphosphorinane (trofosfamide), 2 - (N - (2-chloroethyl) - amino) - 3 - (2 - chloroethyl) - 2-oxo - 1,3,2 - oxazaphosphorinane (ifosfamide), 2 - (N,N - bis - (2 - chloroethyl) - amino) - 2-oxo - 1,3,2 - oxazaphosphorinane (cyclophosphamide) or 2 - (2 - mesyloxyethylamino) - 3-(2 - chloroethyl) - 2 - oxo - 1,3,2 - oxazaphosphorinane (sufosfamide).

8. Pharmaceutical products according to claims 4 to 7 containing the salt of the dithiodi-(alkane sulfonic acid) and the cytostatically active alkylating agent in a weight proportion of at least 0.2:1.

**Revendications**

1. Sels pharmacologiquement acceptables d'un acide dithioalcanique sulfonique de la formule générale

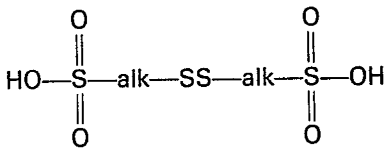

dans laquelle alk est un reste d'alkylène à chaîne droite ou ramifiée comprenant 2 à 6 atomes de carbone, les dits sels étant utilisés pour la thérapie zytostatique à l'aide d'alcoyles.

2. Sels suivant la revendication 1, caractérisés an ce que l'alk est le reste de 1,2-éthylène.

3. En tant que composé suivant les revendications 1 et 2 le sel sodique de l'acide 2,2'-dithiodi-(éthanosulfonique).

4. Produits pharmaceutiques comprenant un sel pharmacologiquement acceptable d'un acide dithiodialcanique sulfonique de la formule générale

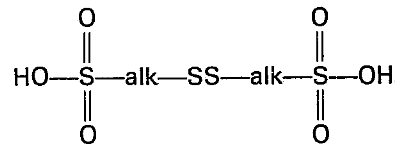

dans laquelle alk est un reste d'alcoylène à chaîne droite ou ramifiée comprenant 2 à 6 atomes de carbone et utilisé comme cytostatique alcoylique.

5. Produits pharmaceutiques suivant la revendication 4, comprenant un sel de l'acide 2,2'-dithiodi-(éthanosulfonique).

6. Produits pharmaceutiques suivant la revendication 5, comprenant le sel disodique de l'acide 2,2'-dithiodi-(éthanosulfonique).

7. Produits pharmaceutiques suivant les revendications 4 à 6, comprenant comme cytostatique alcylique 2 - [N,N - bis - (2 - chloroéthyle) - amino] - 3 - (2 - chloroéthyle) - 2 - oxo-1,3,2 - oxazaphosphorinane (trofosfamide), 2-[N - (2 - chloroéthyle) - amino] - 3 - (2 - chloroéthyle) - 2 - oxo - 1,3,2 - oxazaphosphorinane (ifosfamide), 2 - [N,N - bis - (2 - chloroéthyle)-amino] - 2 - oxo - 1,3,2 - oxazaphosphorinane (cyclophosphamide) ou 2 - (2 - mesyloxyéthylamino) - 3 - (2 - chloroéthyle) - 2 - oxo - 1,3,2-oxazaphosphorinane (sufosfamide).

8. Produits pharmaceutiques suivant les revendications 4 à 7, comprenant le sel de l'acide dithioalcanique sulfonique et le cytostatique alcoylique dans un rapport de poids d'au moins 0,2:1.